# EUROPÄISCHE PATENTSCHRIFT

(11) **EP 2 659 979 B1**
(45) Veröffentlichungstag und Bekanntmachung des Hinweises auf die Patenterteilung: **29.06.2016**
(21) Anmeldenummer: 13166537.4
(22) Anmeldetag: 03.05.2013
(51) Int. Cl.: B01L 9/06

(54) **Haltestruktur mit einer Mehrzahl von gleichzeitig gehaltenen Behältern für medizinische, pharmazeutische oder kosmetische Anwendungen sowie Transport- oder Verpackungsbehälter**
Support structure simultaneously holding of a number of containers for medical, pharmaceutical or cosmetic applications and transport or packaging container
Structure de support portant simultanément une pluralité de récipients pour des applications médicales, pharmaceutiques ou cosmétiques ainsi que récipient de transport ou d'emballage

(30) Priorität: 03.05.2012 DE 102012103896; 03.05.2012 US 201261642142 P
(43) Veröffentlichungstag der Anmeldung: 06.11.2013
(73) Patentinhaber: Schott AG, 55122 Mainz (DE)
(72) Erfinder: Wissner, Kai, 69493 Hirschberg (DE); Pawlowski, Edgar, 55271 Stadecken-Elsheim (DE); Coulon, Joel, 9000 St. Gallen (CH); Dietrich-Ostry, Claudia, 9422 Staad (CH); Lovis, Ralph, Dr.-Ing., 8400 Winterthur (CH)
(74) Vertreter: 2K Patentanwälte Blasberg Kewitz & Reichel

(56) Entgegenhaltungen:
- EP-A2- 0 219 802
- WO-A1-2009/015862
- WO-A1-2011/110872
- AU-B2- 469 958
- DE-A1- 10 345 625
- DE-U1-202006 005 652
- GB-A- 879 397
- GB-A- 2 182 018
- US-A- 6 132 684
- US-A1- 2001 052 476

## Beschreibung

### Gebiet der Erfindung

Die vorliegende Erfindung betrifft allgemein die gleichzeitige Halterung einer Mehrzahl von Behältern zur Aufbewahrung von Substanzen für medizinische, pharmazeutische oder kosmetische Anwendungen, insbesondere von Fläschchen (Vials), insbesondere in solcher Weise, dass diese, während diese in einer hierfür vorgesehenen Haltestruktur gehalten werden, in Abfüll- oder Bearbeitungsanlagen weiter verarbeitet werden können, insbesondere in einem Steriltunnel, einer Abfüllanlage für flüssige medizinische oder pharmazeutische Anwendungen oder einem Gefriertrockenschrank hierfür. Ferner betrifft die vorliegende Erfindung einen Transport- und/oder Verpackungsbehälter mit zumindest einer solchen Haltestruktur sowie optional mit einer integrierten Sensorik und/oder einem Plagiatschutz.

### Hintergrund der Erfindung

Als Behälter zur Aufbewahrung von und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die die Haltestruktur aus dem Transport- und Verpackungsbehälter, die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten werden. Die einzelnen Medikamentenbehälter werden zunächst lose in Aufnahmen, die in der Haltestruktur ausgebildet sind, angeordnet. Anschließend wird die Haltestruktur in den Transport- und Verpackungsbehälter eingesetzt und dieser mit einem gasundurchlässigen Kunststoffschlauch umgeben. Beim anschließenden Evakuieren der so ausgebildeten Verpackungseinheit wird der Kunststoffschlauch aufgrund des in dem Schlauch vorherrschenden Unterdrucks in die Zwischenräume zwischen den Medikamentenbehältern hineingedrückt, was so einerseits zu einer Stabilisierung der Position der Medikamentenbehälter in der Haltestruktur führt und andererseits eine weitere unkontrollierte Kollision von benachbarten Medikamentenbehältern verhindert. Beim Evakuieren und beim Anschließenden Öffnen des Kunststoffschlauchs können jedoch die Medikamentenbehälter seitlich verrutschen, was den Automatisierungsaufwand zur Weiterverarbeitung der Medikamentenbehälter erhöht. Ferner können die Medikamentenbehälter nach dem Öffnen des Kunststoffschlauchs dennoch unkontrolliert kollidieren, was die vorgenannten Nachteile mit sich bringt. Die Medikamentenbehälter können nicht in dem Transport- oder Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1, US 2011/0277419 A1, WO 2012/025549 A1, WO 2011/015896 A1, WO 2012/007056 A1 und WO 2009/015862 A1 offenbart. Zur Weiterverarbeitung müssen die Medikamentenbehälter jedoch stets vereinzelt werden. Eine chargenweise Weiterverarbeitung der Medikamentenbehälter, während diese in einer plattenförmigen Haltestruktur, wie vorstehend ausgeführt, aufgenommen sind, ist nicht möglich.

DE 100 12 575 A1 der Anmelderin offenbart die Klemmung von Spritzenkörpern in einer Trägerplatte aus Kunststoff. Um die Öffnungen der Trägerplatte herum sind umlaufende Vorsprünge ausgebildet, die als Haltemittel wirken und einer weiteren Klemmung dienen. Eine koordinierte Verstellung der Öffnungsweite dieser Öffnungen ist nicht möglich, da die Trägerplatte biegesteif ist.

US 5,950,832 offenbart eine Haltestruktur für Zentrifugenröhrchen, die an ihrem unteren Ende spitz zulaufend sind. Die Haltestruktur weist ein elastisches Teil und ein steifes Teil auf. Der Durchmesser von Bohrungen des elastischen Teils ist kleiner als der Außendurchmesser der Zentrifugenröhrchen, wodurch deren Klemmung ermöglicht wird. Eine koordinierte Verstellung der Öffnungsweite dieser Bohrungen ist nicht offenbart.

WO 2009/015862 A1 offenbart eine Klemmung im Bereich der Seitenwand von Behältern oder an deren Halsabschnitt durch Haltemittel. Eine koordinierte Verstellung der Öffnungsweite dieser Haltemittel ist nicht offenbart.

GB 2478703 A offenbart eine Haltestruktur, die aus zwei Teilen besteht, die aufeinander zugeklappt werden können. Die Aufnahmen der beiden Teile sind zueinander versetzt, sodass die Behälter ineinander geschachtelt angeordnet werden, um die Packungsdichte zu verdoppeln, in der aufgefalteten Stellung jedoch einen guten Zugriff auf die Behälter zu ermöglichen.
DE 20 2006 005652 U1 offenbart eine klappbaren Flaschenkasten für Getränkeflaschen, mit Aufnahmen, deren Seitenwände beim Zusammenklappen des Flaschenkastens durch Ausführen einer Schwerbewegung koordiniert verstellt werden können. Eine Klemmung der Getränkeflaschen im entfalteten Zustand des Flaschenkastens erfolgt nicht.
GB 2 182 018 A offenbart ähnlich wirkende Haltestrukturen. Eine Klemmung der Behälter erfolgt nicht.
WO 2009/015862 A1 offenbart eine Haltestruktur, bei der die Behälter mittels elastischer Haltezungen geklemmt gehalten werden. Die elastischen Haltezungen drücken gegen die verschmälerten Nackenbereiche der Behälter, um diese dort zu klemmen. Eine koordinierte Verstellung von Aufnahmen der Haltestruktur ist nicht offenbart.
Jedenfalls ist ein unmittelbarer Kontakt der Böden der Medikamentenbehälter, insbesondere der Böden von Fläschchen, bei den herkömmlichen Haltestrukturen nicht möglich. Dies erschwert jedoch die Weiterverarbeitung der Medikamentenbehälter insbesondere dann, wenn deren Inhalt einer Gefriertrocknung (auch als Lyophilisation oder Sublimationstrocknung bezeichnet) unterzogen werden soll. Ferner ist eine Weiterverarbeitung der Medikamentenbehälter unmittelbar in den Haltestrukturen nicht möglich, da diese dort entweder starr gehalten werden oder für die Weiterverarbeitung nicht in ausreichendem Maß zugänglich sind, weshalb die Medikamentenbehälter für eine Weiteverarbeitung herkömmlich stets aus den Haltestrukturen entnommen werden müssen.

### Zusammenfassung der Erfindung

Aufgabe der vorliegenden Erfindung ist es, eine Haltestruktur mit einer Merhrzahl von darin gehaltenen zylindrischen Behältern für Substanzen für medizinische oder pharmazeutische Anwendungen, insbesondere von Glasfläschchen, dahingehend weiterzubilden, dass die Behälter einfach und kostengünstig steril verpackt, wieder entpackt und weiterverarbeitet werden können. Gemäß einem bevorzugten weiteren Gesichtspunkt der vorliegenden Erfindung soll eine solche Haltestruktur insbesondere für eine Weiterverarbeitung der Behälter, während diese in der Haltestruktur gehalten werden, ausgelegt sein. Gemäß einem weiteren Gesichtspunkt der vorliegenden Erfindung soll ferner ein entsprechender Transport- und Verpackungsbehälter mit zumindest einer solchen Haltestruktur bereitgestellt werden.

Diese Aufgaben werden gemäß der vorliegenden Erfindung durch eine Haltestruktur mit den Merkmalen nach Anspruch 1 sowie durch einen Transport- und Verpackungsbehälter nach Anspruch 11 gelöst. Weitere vorteilhafte Ausführungsformen sind Gegenstand der rückbezogenen Unteransprüche.

Bei einer Haltestruktur nach der Erfindung sind die Behälter (container) in der Haltestruktur reibschlüssig bzw. geklemmt gehalten. Erfindungsgemäß sind die Aufnahmen von durchgehend ausgebildeten Seitenwänden ausgebildet, wobei Öffnungsweiten von sämtlichen Aufnahmen der Haltestruktur zwischen einer ersten Stellung, in welcher die Behälter frei oder mit geringem Kraftaufwand in die Aufnahmen einführbar sind, und einer zweiten Stellung, in welcher die Behälter geklemmt sind, durch koordiniertes Verstellen der Seitenwände der Aufnahmen verstellbar sind.

Zur kraft- bzw. reibschlüssigen Halterung der zylindrischen Behältern dienen Aufnahmen, die bevorzugt umlaufend ausgebildet sind und die sich in Längsrichtung der Behälter erstrecken. Kraft- bzw. reibschlüssige Verbindungen setzen bekanntermaßen nur eine ausreichende Normal-Kraft auf die miteinander zu verbindenden Flächen voraus. Die gegenseitige Verschiebung zwischen Behälter und Haltestruktur ist also verhindert solange die durch die Haftreibung zwischen Haltestruktur und Behälter bewirkte Gegenkraft nicht überschritten wird. Der Kraft- bzw. Reibschluss ist verloren und die Flächen rutschen aufeinander, wenn die tangential wirkende Last-Kraft größer als die Haftreibungs-Kraft ist. Letzteres ist jedoch bei den vergleichsweise geringen Gewichten der in der Haltestruktur aufzunehmenden Behälter unwahrscheinlich, kann jedoch ausgenutzt werden, um die Behälter, während diese in der Haltestruktur aufbewahrt sind, von einer ersten Stellung axial in eine zweite Stellung zu verschieben, in welcher diese weiterverarbeitet werden können, beispielsweise deren Öffnungen mit einem Stopfen verschlossen werden oder auf den Stopfen ein äußerer Verschluss (beispielsweise Bördelkappe oder Krampe), häufig aus Aluminiumblech, aufgebracht wird.

Der Kraft- bzw. Reibschluss erfolgt zweckmäßig entweder unterhalb des aufgeweiteten oberen Randes der Behälter, d.h. in ihrem verengten Hals- bzw. Nackenbereich unterhalb des oberen Rands, oder im Bereich der zylindrischen Seitenwand. Einer bodenseitigen Abstützung der Behälter bedarf es erfindungsgemäß grundsätzlich nicht, sodass ein Zugriff auf die Unterseiten (Böden) der in der Haltestruktur gehaltenen Behälter grundsätzlich möglich ist. Dies ermöglicht erfindungsgemäß, dass die Behälter, während diese in der Haltestruktur auf genommen sind, weiterverarbeitet werden können. Mit anderen Worten, die Behälter können in den Haltestrukturen chargenweise weiter verarbeitet werden, bleiben jedoch während der Weiterverarbeitung weiterhin zuverlässig und kollisionsfrei in den Haltestrukturen gehalten, was erhebliche Geschwindigkeitsvorteile und Vorteile bei der Automatisierung von Weiterverarbeitungsanlagen ermöglicht und so insgesamt zu noch wirtschaftlicheren und kostengünstigeren Prozessen führt. Die Behälter können insbesondere in der Haltestruktur axial angehoben oder gedreht werden. Die Behälter können gemäß weiteren Ausführungsformen auch in der Haltestruktur lyophilisiert werden, da ein direkter Bodenkontakt zu einem Kühlfinger des Gefriertrockenschranks möglich ist. Böden oder Köpfe der Behälter sind in einfacher Weise auf einer Höhe fixierbar, sodass sämtliche Böden in einer gemeinsam aufgespannten Ebene angeordnet werden können, was den unmittelbaren Kontakt zu flächigen Weiterverarbeitungstationen, insbesondere zu einem Kühlfinger bzw. einer Kühlhorde eines Gefriertrockenschranks ermöglicht. Ferner ist ein unmittelbarer Glas-Glas Kontakt von benachbarten Behältern zuverlässig verhindert, was Abrieb und Verunreinigungen innerhalb der Weiterverarbeitungsanlage wirkungsvoll verhinder und so erheblich längeren Laufzeiten und Wartungsintervalle der Anlagen ermöglicht. Ferner können Kratzer oder die Erzeugung von Partikeln auf oder in den Behältern wirkungsvoll verhindert werden.

Die erfindungsgemäße Haltestruktur ermöglicht dabei zweckmäßig die Entnahme der Behälter nach oben oder unten. Da die Position des Kraft- bzw. Reibschlusses zwischen Behälter und Haltestruktur einfach variiert werden kann, ist die erfindungsgemäße Haltestruktur sehr flexibel auch für Behälter mit unterschiedlichen Außenabmessungen einsetzbar, solange eine ausreichende Normalkraft für den Reibschluss gewährleistet werden kann. Insbesondere können die Behälter in der Haltestruktur in axialer Richtung einfach verschoben werden, sodass Behälter mit unterschiedlichen Höhen in derselben Haltestruktur gehalten werden können. Die axiale Verschieblichkeit der Behälter in der Haltestruktur ermöglicht auch einen einfachen Toleranzausgleich.

Die Aufnahmen der Haltestruktur sind von durchgehend ausgebildeten Seitenwänden ausgebildet, was insbesondere bedeuten soll, dass die Seitenwände der Aufnahmen jeweils nicht mehrstückig ausgebildet sind, insbesondere nicht aus zwei oder mehreren relativ zueinander verschieblich gelagerten Seitenwandabschnitten. Die Seitenwände sind vielmehr plattenförmig und einstückig ausgebildet, was nicht ausschließen soll, dass die Seitenwände mit einer Beschichtung überzogen sind, etwa mit einer Beschichtung mit hohem Reibungskoeffizienten, um die Reibung zwischen den Seitenwänden der Aufnahmen und den Behälter zu erhöhen und so die Behälter noch wirkungsvoller zu klemmen.

Um die Öffnungsweite bzw. den Reibschluss von sämtlichen Aufnahmen der Haltestruktur durch koordiniertes Verstellen der Seitenwände der Aufnahmen gemeinsam bzw. koordiniert zu verstellen, sind sämtliche Aufnahmen der Haltestruktur bevorzugt miteinander mechanisch gekoppelt, sodass eine Verstellung oder Verformung der Haltestruktur zu einer koordinierten Verstellung der Öffnungsweite sämtlicher Aufnahmen führt. Dabei ist die koordinierte Verstellung so ausgelegt, dass die Seitenwände der Aufnahmen zwischen einer ersten Stellung, in welcher die Behälter mit geringem Kraftaufwand in die Öffnungen oder Aufnahmen der Haltestruktur einführbar sind, und einer zweiten Stellung koordiniert verstellbar sind, in welcher die Behälter in den Öffnungen oder Aufnahmen der Haltestruktur reibschlüssig fixiert sind.

Gemäß weiteren Ausführungsformen können die Behälter, während diese in der Haltestruktur und in dem Transport- und Verpackungsbehälter aufgenommen sind, weiter verarbeitet werden. Geht dabei trotz aller Vorsichtsmaßnahmen dennoch ein Behälter zu Bruch, so führt dies nicht zu einer Verunreinigung der Anlage, da der Bruch aber auch etwaige Wirkstubstanzen oder -lösungen in dem Transport- und Verpackungsbehälter verbleiben.

Gemäß einer weiteren Ausführungsform ist zumindest ein überwiegender Teil der Böden oder unteren Enden der Behälter von einer Unterseite der Haltestruktur her frei zugänglich. Bevorzugt sind die Böden oder unteren Enden der Behälter von keinen Haltestrukturen oder dergleichen abgedeckt. Gemäß weiteren Ausführungsformen kann jedoch ein Haltesteg oder dergleichen am unteren Ende der Öffnungen bzw. Aufnahmen der Haltestruktur vorgesehen sein, um die Böden oder unteren Enden der Behälter abzustützen. Somit kann eine Verstelleinrichtung, beispielsweise ein Verstellfinger, von der Unterseite der Haltestruktur her auf die Böden oder untere Enden der Behälter einwirken, um diese, während diese in der Haltestruktur gehalten sind, axial zu verstellen oder auch zu verdrehen. Beispielsweise können die Behälter so in der Haltestruktur geeignet angehoben und in der angehobenen Stellung und während diese in der Haltestruktur gehalten sind weiter verarbeitet werden, beispielsweise mit einem äußeren Verschluss (Bördelkappe oder Krampe) versehen werden. Für die Verstellung der Position der Behälter in der Haltestruktur kann gemäß weiteren Ausführungsformen die Öffnungsweite der Haltemittel, insbesondere die Öffnungsweite von Öffnungen oder Aufnahmen, der Haltemittel verstellt werden, wie nachfolgend ausgeführt, sodass der Reibschluss bei einer größeren Öffnungsweite aufgehoben oder wesentlich reduziert ist und bei einer geringeren Öffnungsweite zum zuverlässigen Halten der Behälter weiterhin besteht.

Die länglichen Aufnahmen der Haltestruktur zum Halten der Behälter können grundsätzlich einen geschlossenen oder im Wesentlichen geschlossenen Boden aufweisen, worauf die Böden oder untere Enden der Behälter abgestützt sein können. Gemäß bevorzugten Ausführungsformen ist das untere Ende der Aufnahmen jedoch vollständig oder im Wesentlichen vollständig ausgebildet. Eine Abstützung auf einem Boden oder Haltesteg am unteren Ende der Aufnahmen ist nicht unbedingt erforderlich, da erfindungsgemäß die Halterung der Behälter mittels eines Reibschlusses bewerkstelligt ist.

Der Reibschluss wird in derartigen länglichen Aufnahmen bevorzugt durch das Zusammenwirken von Seitenwänden der Aufnahmen, bevorzugt von einander diametrial gegenüberliegenden Seitenwänden, mit Abschnitten der zylindrischen Seitenwand oder des Halsabschnittes der Behälter bewerkstelligt. Hierzu können die Aufnahmen einen vieleckigen Querschnitt aufweisen, insbesondere einen viereckigen oder sechseckigen Querschnitt. Grundsätzlich können die Aufnahmen jedoch auch einen kreisförmigen oder elliptischen Querschnitt aufweisen, etwa wenn Haltestege am unteren Ende der Aufnahmen vorgesehen ist, welche die Seitenwände der Aufnahmen auseinanderspreizen, wenn die Haltestruktur nicht durch Rastschienen oder dergleichen in Form gehalten (gespannt gehalten) wird.

Ein weiterer Gesichtspunkt der vorliegenden Erfindung betrifft ferner einen Transport- und Verpackungsbehälter mit zumindest einer Haltestruktur, wie vorstehend ausgeführt und nachfolgend weiter im Detail offenbart.

Ein weiterer Gesichtspunkt der vorliegenden Erfindung betrifft ferner einen Transport- und Verpackungsbehälter mit Maßnahmen für einen Plagiatsschutz, insbesondere für eine Identifikation und/oder Nachverfolgung, wie nachfolgend ausgeführt.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a - 1d: eine Haltestruktur und einen Transport- und Verpackungsbehälter gemäß einer ersten Ausführungsform der vorliegenden Erfindung;
- Fig. 1e - 1f: in stark vergrößerten Teildarstellungen eine Variante der Haltestruktur gemäß der vorgenannten ersten Ausführungsform;
- Fig. 2a - 2c: eine Haltestruktur gemäß einer zweiten Ausführungsform der vorliegenden Erfindung;
- Fig. 3a - 3c: eine Haltestruktur gemäß einer dritten Ausführungsform der vorliegenden Erfindung;
- Fig. 4a - 4e: eine Haltestruktur gemäß einer vierten Ausführungsform der vorliegenden Erfindung;
- Fig. 5a: eine unmittelbar verpackte Haltestruktur;
- Fig. 5b: eine weitere Haltestruktur, bei der Randabschnitte weggeklappt werden können; und
- Fig. 6a - 6b: Maßnahmen zur Identifikation und/oder Nachverfolgung bei einem Transport- und Verpackungsbehälter gemäß der vorliegenden Erfindung.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur sowie ein Transport- und Verpackungsbehälter, der eine solche Haltestruktur aufnimmt, dienen gemäß der vorliegenden Erfindung, wie nachfolgend beschrieben, der gleichzeitigen Halterung einer Mehrzahl von Behältern zur Aufbewahrung von Substanzen für kosmetische, medizinische oder pharmazeutische Anwendungen in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen.

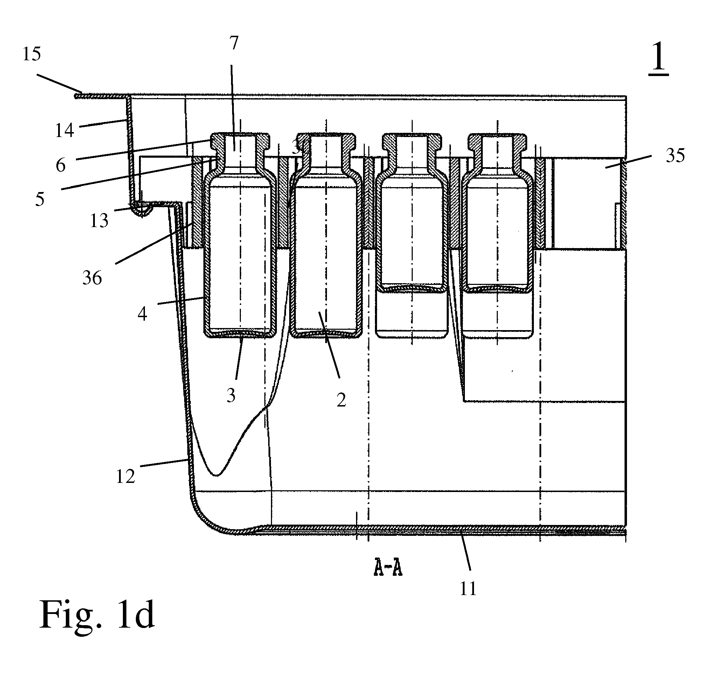

Ein Beispiel für derartige Behälter in Gestalt von Fläschchen (englisch: vial) ist in der Fig. 1d schematisch in einem Längsschnitt dargestellt. Die Fläschchen 2 weisen eine zylindrische Grundform auf, mit einer zylinderförmigen Seitenwand 4 mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Medikamentenbehälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt mit einer ausgezogenen Spitze (Spieß oder Kopf) und einem flachen Boden oder auch mit zwei ausgezogenen Spitzen an beiden Enden.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasfläschchen einen oder mehrere Zehntel Millimeter betragen können. Um solche Fertigungstoleranzen kompensieren zu können und gleichzeitig zu gewährleisten, dass sämtliche Böden 3 oder untere Enden der Behälter in einer Ebene angeordnet werden können, werden die Behälter erfindungsgemäß mittels eines Reibschlusses bzw. einer Klemmung an einer Halterungsstruktur fixiert. Dieser Reibschluss wird entweder im Bereich der zylindrischen Seitenwand 4 oder am unteren, geschlossenen Ende bzw. Boden der Behälter oder gemäß weiteren bevorzugten Ausführungsformen im Bereich des verengten Halsabschnitts 5 realisiert. Im letztgenannten Fall wird jedenfalls die große Mehrzahl der Behälter reibschlüssig im Bereich des verengten Halsabschnitts 5 abgestützt, was jedoch gemäß weiteren Ausführungsformen nicht ausschließen soll, dass bei einzelnen Behältern mit großen Fertigungstoleranzen hinsichtlich ihrer axialen Länge der Übergangsbereich zwischen dem oberen Rand 6 und dem verengten Halsabschnitt 5 nicht auch ausnahmsweise formschlüssig hintergriffen oder abgestützt wird.

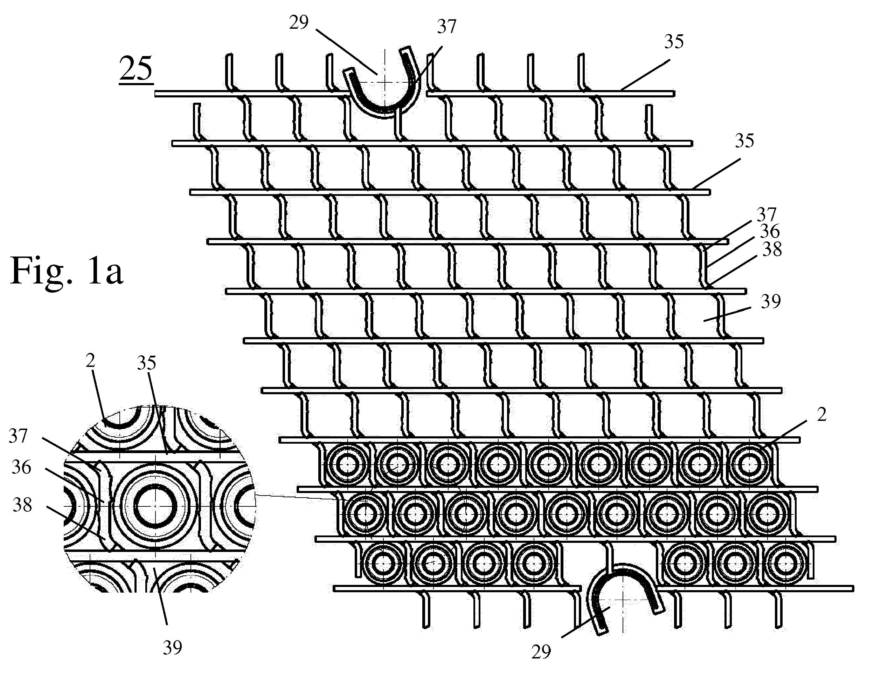

Zum gleichzeitigen Halten einer Mehrzahl von Behältern wird eine Haltestruktur 25 (im Stand der Technik auch als sog. "Nest" bezeichnet) bereitgestellt, die nachfolgend anhand der Figuren 1a bis 1c dargestellt noch genauer beschrieben wird und die aus einem Kunststoff ausgebildet ist, beispielsweise spritzgegossen ist. Die Haltestruktur 25 weist eine Mehrzahl von Aufnahmen 39 auf, die sich in Längsrichtung der aufzunehmenden Behälter 2 erstrecken und miteinander verbunden sind. Bevorzugt sind die Aufnahmen 39 auch mechanisch miteinander gekoppelt. Die Seitenwände der Aufnahmen 39 sind ausreichend flexibel oder aufweitbar, so dass die Behälter 2 von oben oder von unten her in die Aufnahmen 39 eingeführt werden können. Dadurch können eine Mehrzahl von Behältern 2 reibschlüssig fixiert bzw. geklemmt gehalten werden. Aufgrund der Elastizität der Seitenwände der Aufnahmen können auch Fertigungstoleranzen in axialer und/oder radialer Richtung der Behälter ausgeglichen werden, insbesondere bei Medikamentenbehältern aus Glas. Insbesondere können auch Behälter mit unterschiedlichen Durchmessern von ein- und derselben Haltestruktur 25 reibschlüssig fixiert werden.

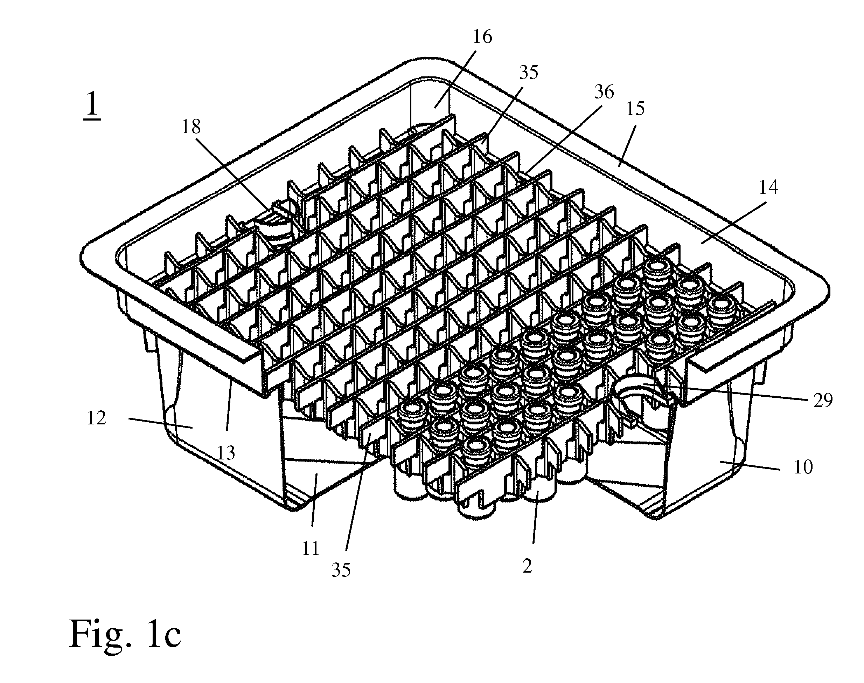

Zum Transport und zur Verpackung einer Haltestruktur im Sinne der vorliegenden Anmeldung mit den darin aufgenommenen Behältern dient ein Transport- und Verpackungsbehälter 10 (im Stand der Technik auch als sog. "Tub" bezeichnet), wie schematisch in der Fig. 1c dargestellt. Gemäß der Fig. 1c ist der Behälter 10 im Wesentlichen kasten- oder wannenförmig ausgebildet und weist einen Boden 11, eine senkrecht von diesem abragende, umlaufend ausgebildete Seitenwand 12, eine von dieser im Wesentlichen rechtwinklig abragende Stufe 13, eine umlaufend ausgebildete obere Seitenwand 14 und einen oberen Rand 15 auf, der flanschartig ausgebildet ist. Die Ecken 16 des Behälters 10 sind zweckmäßig abgerundet ausgebildet. Die obere Seitenwand 14 kann unter einem geringen Neigungswinkel zur Senkrechten auf den Boden 11 geneigt ausgebildet sein, um das Einführen der Haltestruktur 25 zu erleichtern. Ein derartiger Transport- und Verpackungsbehälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle der in dem Transport- und Verpackungsbehälter 10 aufgenommenen Haltestruktur 25 und der von dieser gehaltenen Behälter 2 zu ermöglichen.

Zur Aufnahme der Haltestruktur 25 in dem Transport- und Verpackungsbehälter 10 kann die Haltestruktur 25 von einem Halterahmen umgeben sein, der einen geschlossen ausgebildeten Randsteg aufweist. Die in der Fig. 1c dargestellte Haltestruktur 25 kann grundsätzlich auch in einen Halterahmen eingespannt sein bzw. von diesem entlang des Rands geklemmt seinoder kann einstückig mit einem solchen Halterahmen ausgebildet sein. Zur zuverlässigen Positionierung der Haltestruktur 25 in dem Behälter 10 können die Haltestruktur 25 und der Behälter 10 miteinander zusammenwirkende Positionierungsgebilde aufweisen, die insbesondere formschlüssig zusammenwirken. So können an geeigneter Stelle, insbesondere auf der Stufe 13 oder auf Abstützflächen 18 des Behälters 10 Positionierungsgebilde in Form von Vorsprüngen oder Aussparungen bzw. Vertiefungen ausgebildet sein, die mit korrespondierend ausgebildeten Aussparungen bzw. Vertiefungen oder Vorsprüngen der Haltestruktur formschlüssig zusammenwirken, um die Haltestruktur 25 präzise in dem Transportbehälter 10 zu positionieren. Zu diesem Zweck können auf der Stufe 13 mehrere zapfenartige Vorsprünge ausgebildet, die in korrespondierend ausgebildete Zentrieröffnungen in der Haltestruktur eingreifen. Gemäß der Fig. 1c ist die Stufe 13 des Transportbehälters 10 als umlaufende, ebene Abstützfläche ausgebildet, auf welcher die Haltestruktur 25 unmittelbar aufliegt. Gemäß weiteren Ausführungsformen können an den Seitenwänden 12 des Transportbehälters 10 auch weitere Abstützflächen oder Abstützelemente ausgebildet sein, insbesondere in Form von Vorsprüngen, wie nachfolgend ausgeführt. Auf diese Weise kann die Haltestruktur 25 präzise in dem Transportbehälter 10 positioniert werden und die Mehrzahl von Behältern 2 auf diese Weise in einer regelmäßigen Anordnung und an präzise definierten Positionen in einem Transportbehälter 10 mit standardisierten Abmessungen angeordnet und gehalten werden. Insbesondere kann auf diese Weise gewährleistet werden, dass sämtliche Böden der Behälter in einer gemeinsam aufgespannten Ebene parallel zum Boden 11 oder zum oberen Rand 15 des Transportbehälters 10 angeordnet sind.

Wenngleich in der Fig. 1c der Boden 11 des Transportbehälters 10 als geschlossen und einstückig mit der Seitenwand 12 ausgebildet dargestellt ist, kann das untere Ende des Transportbehälters 10 auch in der Art des oberen Endes geöffnet ausgebildet sein, insbesondere mit einem flanschartigen unteren Rand in der Art des oberen Rands 15 versehen sein, so dass die Böden der Behälter von der Unterseite des Transportbehälters 10 her frei zugänglich sind, beispielsweise für Verarbeitungsschritte in einem Steriltunnel oder in einem Gefriertrockenschrank, wie nachfolgend näher erläutert.

Wie in der Fig. 1c dargestellt, sind in der regelmäßigen Anordnung gemäß der Fig. 1c die Mehrzahl von Behältern 2 entlang von zwei zueinander orthogonalen Richtungen in einer Ebene unter vorbestimmten konstanten Abständen zueinander verteilt angeordnet. Grundsätzlich sind auch weitere regelmäßige Anordnungen denkbar, beispielsweise können zueinander benachbarte Reihen bzw. Spalten von Behältern 2 auch um eine vorbestimmte Länge versetzt zueinander angeordnet sein, und zwar in einer wiederkehrenden Anordnung mit vorbestimmter Periodizität. Somit können automatisierte Fertigungsanlagen die Behälter 2 bei Übergabe an eine Bearbeitungsstation an präzise vorgebbaren Positionen erwarten, was den Automatisierungsaufwand erheblich verringert. Wie nachfolgend näher erläutert, können erfindungsgemäß die Behälter 2 auch innerhalb der Haltestruktur 25 oder des Transportbehälters 10 gemeinsam weiterverarbeitet werden, insbesondere auch in einem Steriltunnel oder einem Gefriertrockenschrank.

Damit die Haltestruktur 25 leicht in den Behälter 10 eingesetzt und aus diesem entnommen werden kann, sind an zwei Längsseiten der Haltestruktur 25 Zugriffsöffnungen 29 ausgebildet, über welche Greifarme oder dergleichen die Haltestruktur 25 greifen können. Wie in der Fig. 1c erkennbar, sind die Zugriffsöffnungen 29 um eine Reihe versetzt zueinander angeordnet, was eine eineindeutige Positionierung der Haltestruktur 25 in dem Transportbehälter 10 weiter erleichtert.

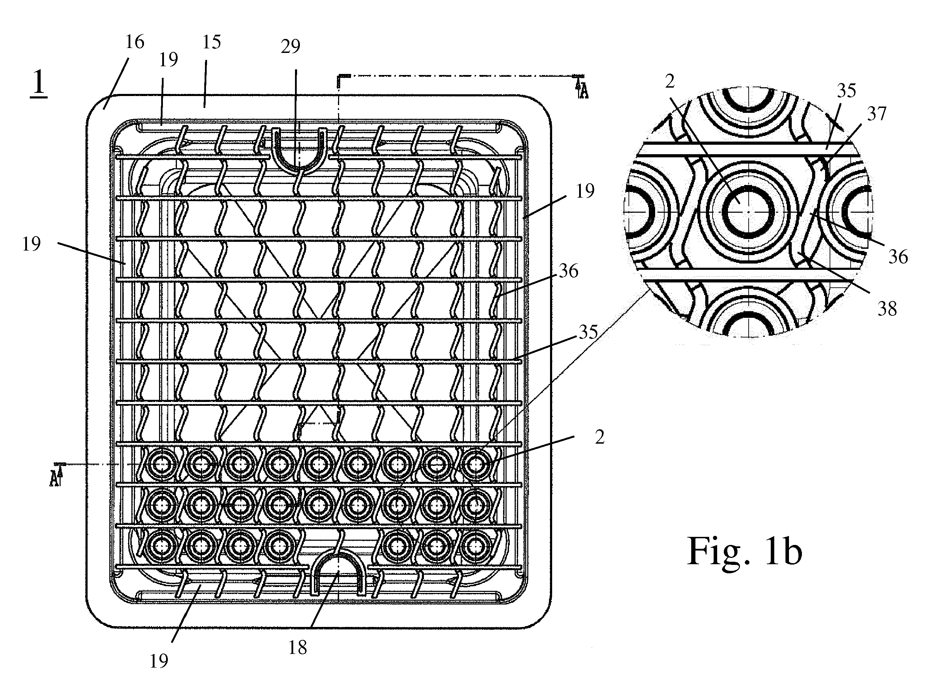

Die Fig. 1b zeigt eine Draufsicht auf die Verpackungseinheit 1 gemäß der Fig. 1c. Die Fig. 1d zeigt einen Längsschnitt entlang der Linie A - A gemäß der Fig. 1c.

Die Fig. 1a zeigt eine Haltestruktur 25 gemäß einer ersten Ausführungsform gemäß der vorliegenden Erfindung. Diese weist eine Mehrzahl von parallel zueinander verlaufenden Querstegen 35 auf, die über unter regelmäßigen Abständen zueinander angeordnete S-förmige Verbindungsstege 36 miteinander verbunden sind, die im Wesentlichen rechtwinklig zu den Querstegen 35 verlaufen. Genauer gesagt sind die Verbindungsstege 36 über in entgegengesetzte Richtungen gekrümmte vordere bzw. hintere Enden 37, 38 mit den Querstegen 35 verbunden. Die Verbindungsstege 36 sind aus einem Kunststoff hergestellt, bevorzugt aus einem flexiblen Kunststoff. Die Querstege 35 weisen bevorzugt eine größere Steifigkeit als die Verbindungsstege 36 auf. Aufgrund der S-förmigen Formgebung der Verbindungsstege 36 sind die Querstege 35 jeweils zueinander in deren Längsrichtung um eine konstante Distanz versetzt, so dass die Haltestruktur 35 insgesamt als Parallelogramm ausgebildet ist, mit einer Basis im Bereich des unteren Rands der in der Fig. 1a dargestellten Haltestruktur 25 und zwei dazu unter einem spitzen Winkel geneigt verlaufenden imaginären Randlinien, welche die vorderen Enden der Querstege 35 miteinander verbinden. In der im rechten Bildteil der Fig. 1a dargestellten entspannten Ausgangsposition können die Behälter 2 frei und ohne Berührung mit den Stegen 35, 36 oder jedenfalls mit nur geringem Kraftaufwand in die von den Stegen 35, 36 ausgebildeten länglichen Halteaufnahmen 39 eingeführt werden. Die Halteaufnahmen 39 weisen im Wesentlichen einen quadratischen Querschnitt auf, der so auf den Durchmesser der Behälter 2 abgestimmt ist, dass diese darin in einer zweiten Stellung der Haltestruktur 25 mit ausreichender Kraft reibschlüssig fixiert, insbesondere geklemmt werden können.

Um die Haltestruktur 25 aus der in der Fig. 1a dargestellten ersten Stellung in die in der in Fig. 1b dargestellte zweite Stellung zu überführen, werden die Querstege 35 jeweils in deren Längsrichtung so verschoben, dass schließlich die in der Fig. 1b dargestellte rechteckförmige oder quadratische Haltestruktur 25 ausgebildet ist. Wie dem Vergleich der Fig. 1a und 1b entnommen werden kann, verbiegen sich hierzu die Verbindungsstege 36 geringfügig. Die zweite Stellung gemäß der Fig. 1b kann durch das Zusammenwirken der in der Haltestruktur 25 ausgebildeten Zugriffsöffnungen 29 mit korrespondierend ausgebildeten Gegenelementen des Behälters 10 fixiert werden oder auch durch das Zusammenwirken von Zentrieröffnungen mit korrespondierend ausgebildeten Zentrierzapfen eines nicht dargestellten Halterahmens, welcher die Haltestruktur 25 aufnimmt.

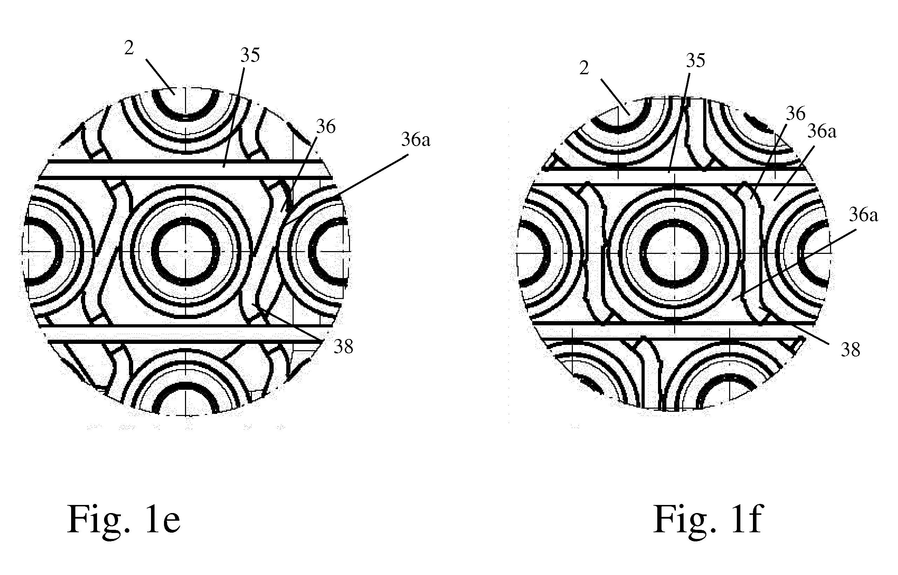

Der vergrößerten Teildarstellung in der Fig. 1 a kann man entnehmen, dass die Behälter 2 in der ersten Stellung gemäß der Fig. 1a lose in den Halteaufnahmen 39 aufgenommen sind. Der vergrößerten Teildarstellung gemäß der Fig. 1b kann man entnehmen, dass die Behälter 2 in der zweiten Stellung gemäß der Fig. 1b von zentralen Abschnitten der Verbindungsstege 36 reibschlüssig fixiert, insbesondere geklemmt, werden. Zu den Querstegen 35 kann weiterhin ein gewisses Spiel bestehen, das jedoch bevorzugt minimal oder verschwindend ist.

Die Fig. 1c zeigt die so ausgebildete Verpackungseinheit 1 in einem perspektivischen Teilschnitt, wobei erkennbar ist, dass bei dieser Ausführungsform die Querstege 35 unmittelbar auf der von der Stufe 13 des Behälters 10 ausgebildeten Abstützfläche abgestützt sind. Die reibschlüssige Fixierung der Behälter ist in dem Teilschnitt gemäß der Fig. 1d erkennbar.

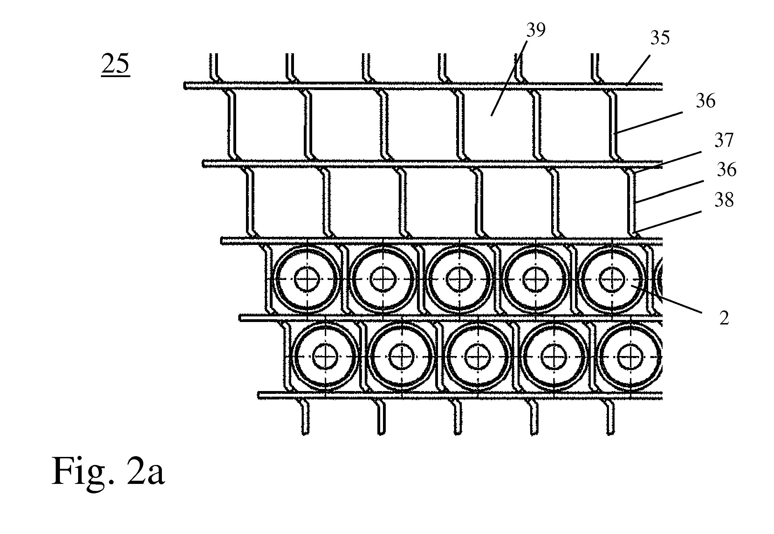

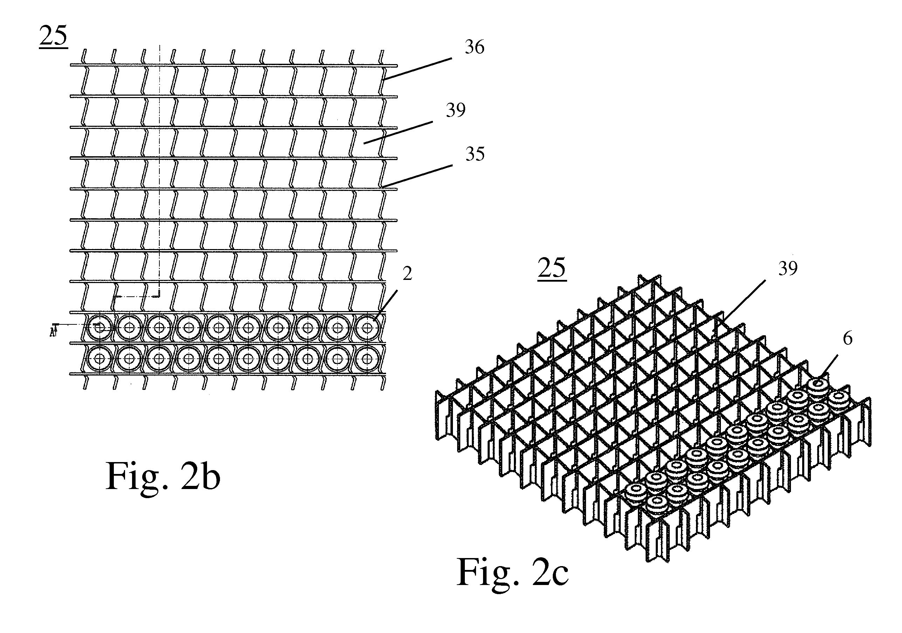

Die Figuren 2a bis 2c zeigen die Haltestruktur gemäß einer weiteren Ausführungsform in einer schematischen Draufsicht und einer Perspektivansicht in der vorgenannten zweiten Stellung.

Wie vorstehend ausgeführt, werden bei der vorstehend beschriebenen ersten Ausführungsform sämtliche Seitenwände der Aufnahmen 39 beim Verschieben der Querstege 35 koordiniert, d.h. gemeinsam, von der ersten Stellung in die zweite Stellung verstellt, und zwar durch Verschwenken des oberen Endes der Haltestruktur 25 (vgl. Fig. 1a) relativ zu der Basis am unteren Ende des in der Fig. 1a dargestellten Parallelogramms. In der vorgenannten ersten Stellung können die Behälter somit in die Haltestruktur eingeführt und präzise positioniert werden, insbesondere auf einem vorbestimmten Höhenniveau so angeordnet werden, dass sämtliche Böden der Behälter bündig und in einer gemeinsamen Ebene angeordnet sind. Durch koordiniertes Verstellen der Haltestruktur in die vorgenannte zweite Stellung werden dann sämtliche Behälter gleichzeitig reibschlüssig fixiert und präzise in einer regelmäßigen Anordnung positioniert. Die Haltestruktur gemäß den Fig. 1a bis 1c ist bevorzugt einstückig aus einem Kunststoff ausgebildet.

Die Figuren 1e und 1f zeigen in stark vergrößerten Teildarstellungen eine weitere Variante der Haltestruktur gemäß den Figuren 1a-1d. Dabei zeigt die Fig. 1e den Bereich einer Auf nahme der Haltestruktur in der vorgenannten zweiten Stellung, in der die Behälter 2 in den Aufnahmen der Haltestruktur gehalten sind. Abweichend zu den Figuren 1a-1d sind bei dieser Ausführungsform an den Verbindungsstege 36 auf den beiden Seiten jeweils ein konkav ausgebildeter Abschnitt 36a ausgebildet, wobei der Krümmungsradius der beiden konkaven Aufnahmen der Abschnitte 36a auf den Radius der Behälter 2 abgestimmt ist. In der zweiten Stellung gemäß der Fig. 1e, in welcher die Verbindungsstege 36 sich geneigt zu den Querstegen 35 erstrecken, schmiegen sich die konkaven Aufnahmen 36a an die zylindrische Seitenwand der Behälter 2 an, sodass die Behälter noch zuverlässiger und kontrollierter gehalten werden können. In der ersten Stellung gemäß der Fig. 1f, in welcher die Verbindungsstege 36 sich senkrecht zu den Querstegen 35 erstrecken, sind die konkaven Aufnahmen 36a nicht mehr gegenüberliegend zur zylindrischen Seitenwand der Behälter 2 angeordnet, sodass die Behälter ungehindert, jedenfalls mit deutlich reduziertem Kraftaufwand, in die von den Stegen 35, 36 ausgebildeten Aufnahmen eingeführt und aus diesen herausgenommen werden können. Idealerweise liegen die Stege 35, 36 in der ersten Stellung gemäß der Fig. 1f überhaupt nicht an den Seitenwänden der Behälter 2 an.

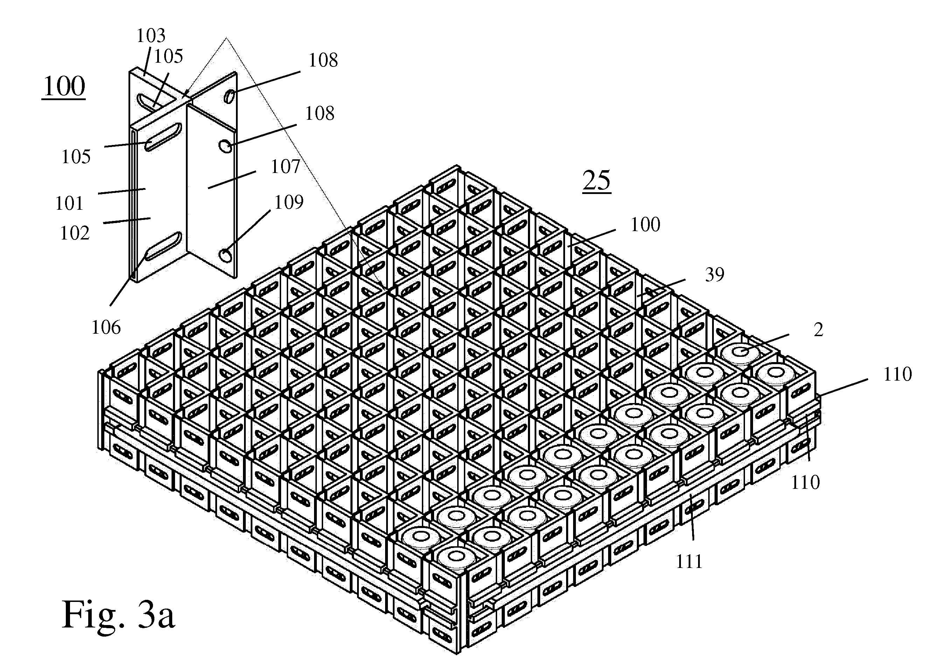

Eine ähnlich wirkende Haltestruktur, wie vorstehend anhand der Figuren 1a-1f beschreiben, kann auch aus einer Mehrzahl identischer Grundeinheiten zusammengesteckt werden, wie nachfolgend anhand der Fig. 3a bis 3c beschrieben.

Gemäß dem oberen Bildteil der Fig. 3a weist eine Grundeinheit 100 sich kreuzförmig schneidende Seitenwände 101, 103, 107 mit identischen Schenkellängen auf. Zwei der Seitenwände, nämlich die Seitenwände 101, 103 sind geschlitzt ausgebildet, mit einem Längsschlitz 102, der sich im Wesentlichen über die gesamte Höhe der Seitenwände 101, 103 erstreckt. In den Seitenwänden 101, 103 sind ein oberes Langloch 105 und ein unteres, parallel dazu verlaufendes Langloch 106 ausgebildet. Die Dicke der Seitenwände 107 ist auf die Breite des Längsschlitzes 102 abgestimmt, so dass diese darin aufgenommen werden können. Auf den Seitenwänden 107 sind zylindrische Vorsprünge 108, 109 ausgebildet, die, wenn die Seitenwände 107 in dem zugeordneten Längsschnitz 102 eingeführt sind, in dem oberen bzw. unteren Langloch 105, 106 geführt sind. Eine Mehrzahl solcher Grundeinheiten 100 wird zu einer rechteckförmigen Haltestruktur 25 zusammengesteckt, wie im rechten unteren Bildteil der Fig. 3a dargestellt.

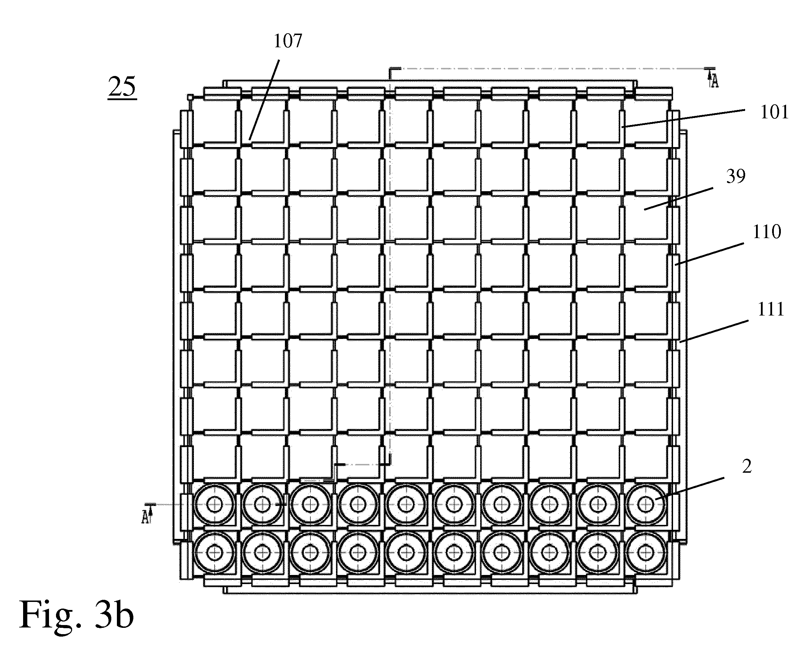

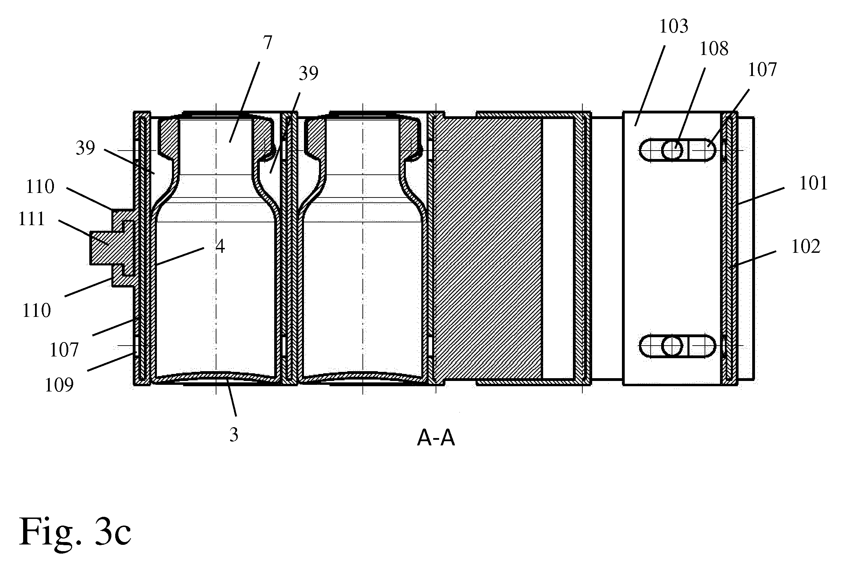

Die Fig. 3b zeigt die Haltestruktur 25 gemäß der Fig. 3a in einer schematischen Draufsicht. Die Seitenwände 101 der Grundeinheiten dienen als Führungsplatten für die darin aufgenommenen Rastplatten 107 der Grundeinheiten. Aufgrund des Zusammenwirkens der Vorsprünge 108, 109 mit den zugeordneten Langlöchern 105, 106 sind die Grundeinheiten in der Ebene der Haltestruktur 25 verschieblich gelagert. Durch das Zusammenwirken der Grundeinheiten werden somit eine Mehrzahl von im Wesentlichen quadratischen oder rechteckförmigen, länglichen Aufnahmen 39 ausgebildet, in die die Behälter 2 von oben oder von unten her eingeführt werden können. Zum Einführen der Behälter werden die Grundeinheiten so auseinandergespreizt, dass die Öffnungsweiten der Aufnahmen 39 ein ungehindertes Einführen oder jedenfalls ein Einführen der Behälter mit nur geringem Kraftaufwand ermöglichen. Anschließend können sämtliche Grundeinheiten so koordiniert gegeneinander gedrückt werden, dass die in den Aufnahmen 39 aufgenommenen Behälter 2 schließlich mit ausreichender Kraft reibschlüssig fixiert, insbesondere geklemmt werden. Auch bei dieser Ausführungsform kann erreicht werden, dass sämtliche Böden der Behälter 2 von der Unterseite der Haltestruktur 25 her frei zugänglich sind und gemeinsam in einer Ebene angeordnet sind. Die Fig. 3c zeigt die Haltestruktur 25 gemäß der Fig. 3b in einem Teil-Längsschnitt entlang der Linie A-A gemäß der Fig. 3b.

Die rechteckförmige Grundform der Haltestruktur 25 wird bei diesem Ausführungsbeispiel durch im Profil L-förmige Formschlusselemente 110 bewerkstelligt, die auf den Außenwänden der äußeren Grundeinheiten angeordnet sind, so dass eine im Wesentlichen U-förmige Aufnahme mit einem Längsschlitz in der Basis ausgebildet ist, in welchen die mit einem korrespondierenden T-förmigen Profil versehene Rastschiene 111 eingreift. Mittels der Rastschiene 111 kann auch eine Fixierung der Position sämtlicher Grundeinheiten 100 erzielt werden, beispielsweise durch Festschrauben oder Festklemmen der Rastschiene 111 jedenfalls an den vordersten und hintersten Grundeinheiten 100 einer Spalte bzw. Reihe der matrixförmigen Haltestruktur 25.

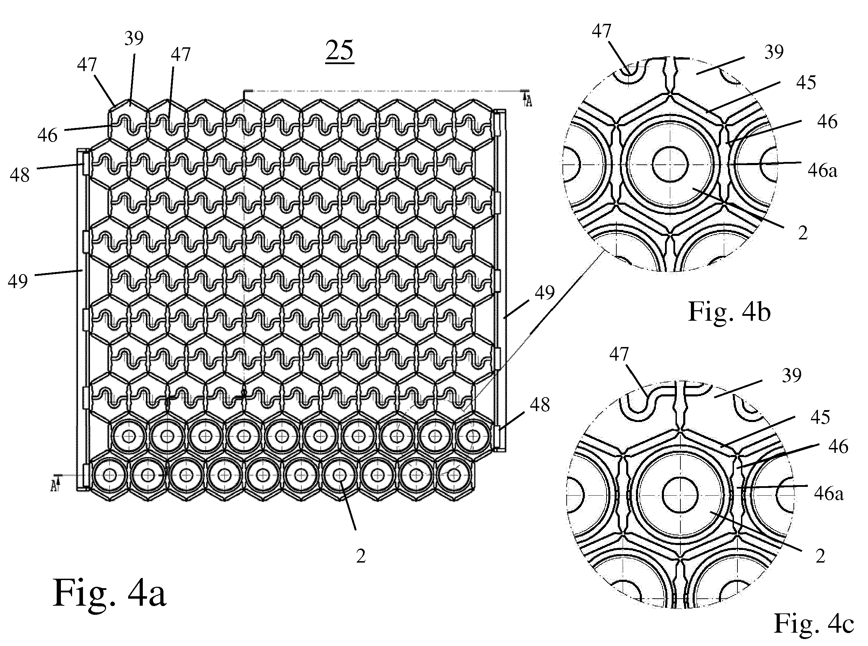

Die Figuren 4a bis 4e zeigen eine Haltestruktur 25 gemäß einem weiteren Ausführungsbeispiel. Diese ist von einer Mehrzahl von hexagonalen Aufnahmen 39 zur Aufnahme von Behälter 2 ausgebildet, wobei die Reihen der hexagonalen Aufnahmen alternierend versetzt zueinander angeordnet sind. Die hexagonalen Aufnahmen 39 werden von zwei Paaren von unter einem stumpfen Winkel aufeinander zu laufenden Seitenwänden 45 ausgebildet, die über flexible Klemmstege 46 miteinander verbunden sind. Wie man der Fig. 4b entnehmen kann, weisen die flexiblen Klemmstege 46 einen jeweils konkav ausgebildeten Halteabschnitt 46a auf, wobei die Verbindungsstege 47 spiegelsymmetrisch ausgebildet sind, so dass die konkaven Einbauchungen auf den beiden Seiten der Klemmstege 46 den Seitenwänden der zugeordneten Behälter 2 zugewandt sind. Am unteren Ende der Halteaufnahmen 39 verbinden wellenlinienförmige Stege 47 die beiden Klemmstege 46a miteinander. Die Stege 47 können als Haltestege zur Abstützung der in den Aufnahmen 39 aufgenommenen Behältern 2 dienen. Alternativ können die Stege 47 jeweils einander gegenüberliegende Seitenwände der Aufnahmen 39 elastisch gegeneinander vorspannen, sodass ohne weiteres Behälter mit unterschiedlichen Durchmessern geklemmt werden können. Die Haltestruktur 25 gemäß der Fig. 4a kann grundsätzlich einstückig aus einem Kunststoff spritzgegossen sein. Die Haltestruktur 25 kann gemäß weiteren Ausführungsformen jedoch auch aus einer Mehrzahl von wabenförmigen Grundeinheiten zusammengesetzt werden, die jeweils einzeln hergestellt und in der dargestellten Anordnung zusammengesetzt werden und in dieser Anordnung von Rastschienen 49, wie nachfolgend beschrieben, zusammengehalten werden.

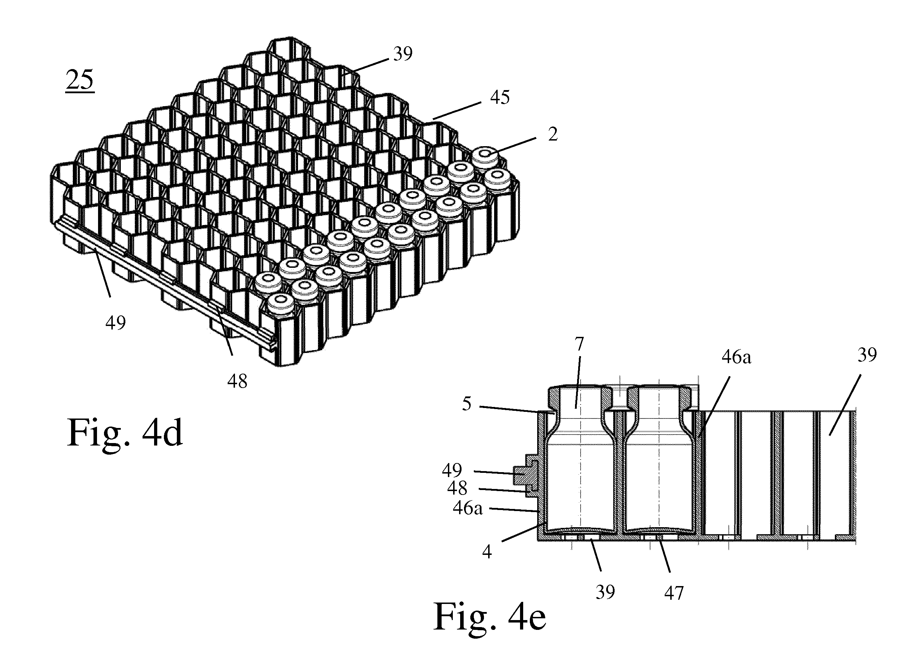

Die wellenlinienförmigen Stege 47 drücken die Aufnahmen 39 der Haltestruktur 25 in der Darstellung gemäß der Fig. 4a seitlich auseinander, so dass die Klemmstege 46 mit ihren Einbauchungen 46a in der in der Fig. 4c dargestellten ersten Stellung gegen die Seitenumfangswand der Behälter 2 gedrückt werden, sodass die Behälter 2 mit ausreichenden Haltekräften in den Aufnahmen 39 aufgenommen sind, wie in dem Einsatz gemäß der Fig. 4b dargestellt. Um diese Haltestruktur 25 ausgehend von der Stellung gemäß der Fig. 4b in die in der Fig. 4c dargestellte zweite Stellung zu überführen, in welcher die Klemmstege 46 mit ihren Einbauchungen 46a die Seitenumfangswände der Behälter 2 nicht mehr klemmen oder allenfalls nur noch mit einer geringen Kraft, sodass die Behälter aus den Aufnahmen ohne größeren Kraftaufwand entnommen bzw. darin verstellt werden können, muss die Haltestruktur 25 in der Darstellung gemäß der Fig. 4a in Querrichtung auseinandergezogen werden. Zu diesem Zweck sind an den Außenwänden der äußersten hexagonalen Aufnahmen 39 im Profil L-förmige Formschlusselemente 48 vorgesehen sind, die T-förmige Aufnahmen ausbilden, in die eine korrespondierend ausgebildete Rastschiene 49 eingeschoben ist. Durch Ziehen an diesen Rastschienen in Querrichtung kann die Haltestruktur insgesamt aufgespreizt werden, unter gleichzeitiger Aufweitung sämtlicher Aufnahmen 39. Je nachdem, wie stark seitlich an den Rastschienen 49 gezogen wird, können die Aufnahmen 39 geeignet aufgeweitet werden. Die in der Fig. 4d gezeigten Rastschienen 49 können in einen seitlichen Halterahmen eingehängt oder auf einer Abstützfläche eines Transport- und Verpackungsbehälters abgestützt werden. Durch Stauchen der Halteaufnahmen 39 können somit insgesamt die Öffnungsweiten der Aufnahmen 39 koordiniert von der ersten Stellung gemäß der Fig. 4c in die zweite Stellung gemäß der Fig. 4b überführt werden, in welcher die Behälter 2 an vorbestimmten Positionen reibschlüssig fixiert sind. Die Höhenlage der Behälter 2 wird dabei aufgrund der Auflage der Böden der Behälter 2 auf den Stegen 47 im Wesentlichen durch die Stege 47 festgelegt. Wie man der Schnittdarstellung gemäß der Fig. 4e entnehmen kann, ist auch bei dieser Ausführungsform der größte Teil des Bodens der Behälter 2 von unten her frei zugänglich, beispielsweise für eine mechanische Verstelleinrichtung.

Während vorstehend dargelegt wurde, dass die Haltestruktur auf der von der Stufe 13 nahe dem oberen Rand ausgebildeten Auflagefläche abgestützt ist, kann diese grundsätzlich auch unmittelbar auf dem Boden 11 des Transportbehälters 10 abgestützt sein. Dies funktioniert grundsätzlich bei sämtlichen Haltestrukturen, die über eine gewisse axiale Länge verfügen, sei es dass die Böden der Behälter 2 unmittelbar auf dem Boden 11 des Behälters 10 aufliegen oder auf Böden der länglichen Aufnahmen 39 der Haltestruktur 25.

Während bei den vorherigen Ausführungsbeispielen dargelegt wurde, dass die Behälter aufrecht und mit ihrem offenen Ende zum oberen Ende des Transport- und Verpackungsbehälters hin angeordnet sind, können die Behälter grundsätzlich auch gewendet, d.h. mit ihrem offenen Ende hin zum Boden des Transport- und Verpackungsbehälters zeigend, angeordnet sein.

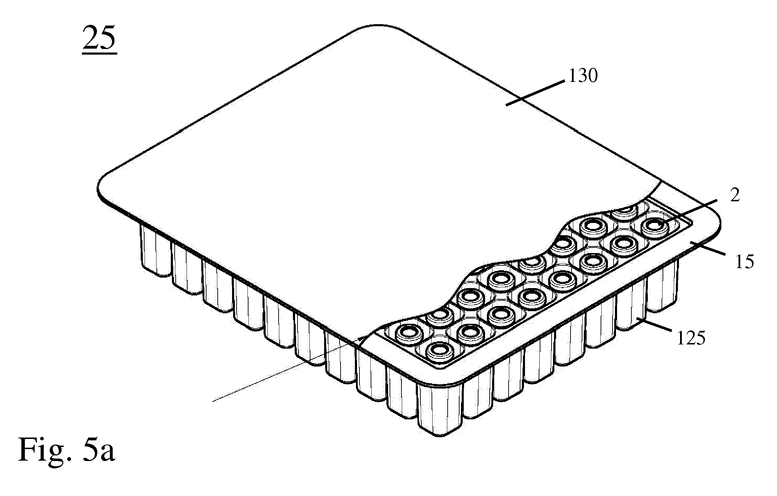

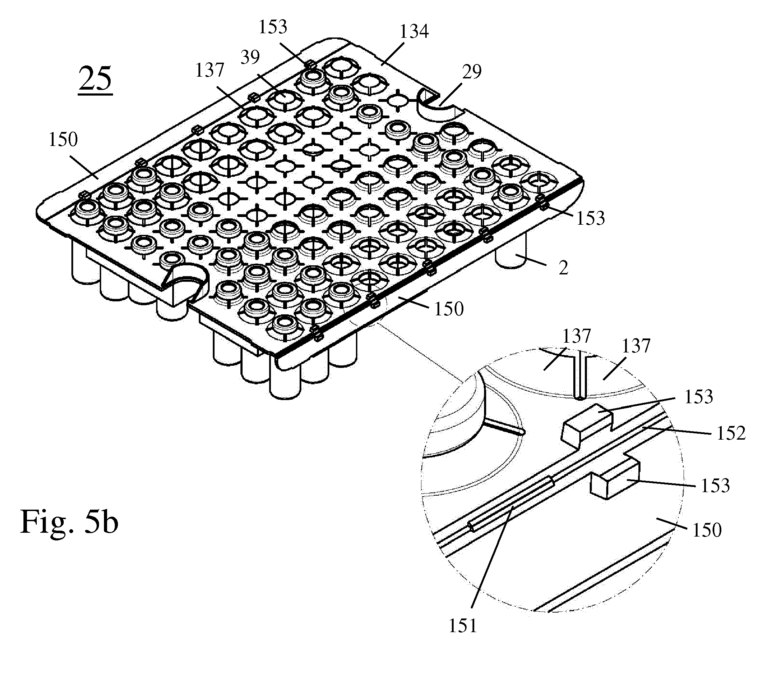

Die Oberseite oder die Ober- und Unterseite einer Haltestruktur 25 gemäß der vorliegenden Erfindung oder auch eines Transport- und Verpackungsbehälters 1 gemäß der vorliegenden Erfindung kann bzw. können von einer sterilen, gasdurchlässigen Schutzfolie überzogen sein, die aufgeklebt und bei Bedarf abziehbar ist. Dies ist beispielhaft für eine Haltestruktur 25 in der Fig. 5a dargestellt, für die dargestellt ist, dass die Folie 130 auf den oberen Rand 15 aufgeklebt ist, wobei weitere Einzelheiten der (in diesem Fall formschlüssigen) Halterung der Behälter 2 hier nicht weiter diskutiert werden sollen. Bei der Schutzfolie kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Behälter 2 durch die Folie 130 hindurch ermöglicht.

Bei sämtlichen Ausführungsformen der Erfindung können dem Kunststoff der Haltestruktur (des Trägers) und/oder des Transport- und Verpackungsbehälters antimikrobiell wirkende Pulver beigemischt sein. Antimikrobielle wirkende Pulver mit Atomen oder Ionen, wie beispielsweise Ag, Zn, Cu, Ce, Te oder I, sind aufgrund ihrer bioziden, bakterioziden, und fungiziden Wirkung als Zuschlagstoff oder Füllstoff für die unterschiedlichsten Zwecke geeignet. Die biozide Wirkung tritt beispielsweise gegenüber Bazillen, Pilzen, Viren, Hefen, Algen und anderen Kleinlebewesen ein. Als biozid wirkende Pulver können dem Kunststoff der Haltestruktur (des Trägers) und/oder des Transport- und Verpackungsbehälters insbesondere auch Glaspulver zugegeben werden, die Ag₂0, CuO, Cu₂0, Te0₂, ZnO, Ce0₂ und I enthalten. Für die Anwendung in Kunststoffen bleiben die mechanischen und optischen Eigenschaften weitgehend unverändert. Die Einbringung der Pulver kann hierbei entweder bereits als Nanopulver beispielsweise den Kunststoffpulvermischungen beim Spritzguss oder in Lacken bei Tiefziehprozessen mit eingebracht werden. Eine antimikrobielle Wirkung kann auch erzielt werden, wenn der Kunststoff mit einer antimikrobiellen Beschichtung versehen ist, welche die vorgenannten Pulver enthalten.

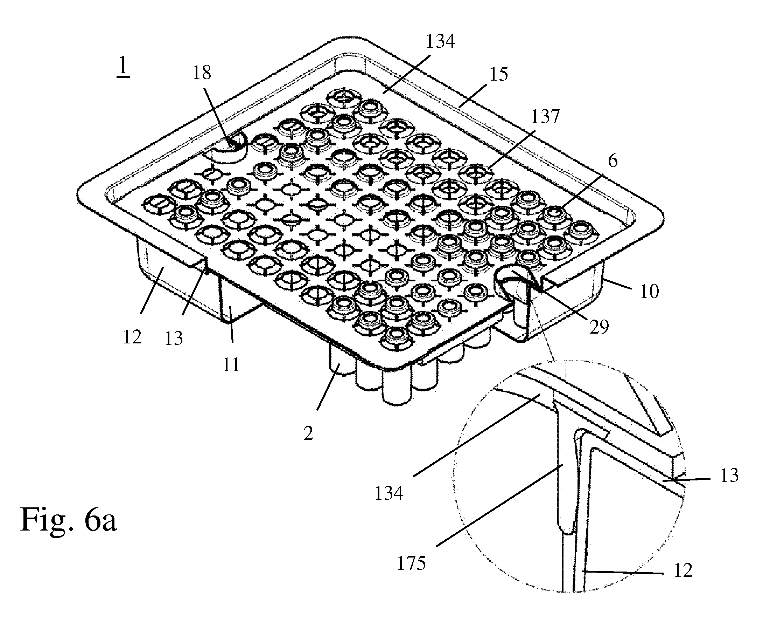

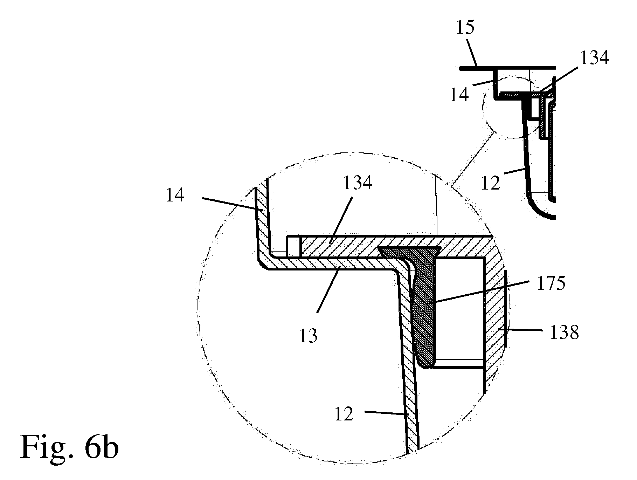

Die Figuren 6a und 6b zeigen eine weitere Ausführungsvariante für eine Verpackungseinheit 1 mit einer in dem Transport- und Verpackungsbehälter 10 aufgenommenen Halteplatte 134, in welcher eine Mehrzahl von Behältern 2 mittels Ringelementen 137 reibschlüssig fixiert sind. Die Verpackungseinheit 1 weist Vorkehrungen zur Identifikation und/oder Nachverfolgung wie folgt auf: Wie in dem vergrößerten Einsatz der Fig. 15a dargestellt, ist in dem Bereich der Zugriffsaussparung 29 zwischen die Halteplatte 134 und die Seitenwand 12 und/oder die Stufe 13 des Behälters 10 ein elektronisch drahtlos (wireless) auslesbarer RFID-Chip oder RuBee-Chip 175 angeordnet (ein RuBee Chip funkt auf Frequenzen, die Metall- und Wasser durchdringen können), der durch die Seitenwände der Verpackungseinheit 1 hindurch berührungslos ausgelesen werden kann und auf Abfrage Information bezüglich der Identität, wichtige Produkteigenschaften (Hersteller, Inhalt, Herstellungsdatum, Verfallsdatum, ...) etc. ausgibt. Der Chip 175 kann in die Verpackungseinheit 1 an geeigneter Stelle eingeklebt sein, auch an anderer Stelle als in der Figur dargestellt. Der Chip 175 kann so angeordnet sein, dass im Falle eines Öffnens der Verpackungseinheit 1 oder eines Herausnehmens der Halteplatte 134 aus der Verpackungseinheit 1 heraus der Chip 175 zerstört wird, beispielsweise zerrissen oder funktionsunfähig wird. Aufgrund der fehlenden Antwort des Chips 175 auf eine Funkabfrage hin steht somit eine Information zur Verfügung, die anzeigt, dass die Verpackungseinheit seit der vorherigen Verpackung in irgendeiner Weise manipuliert worden sein muss. Denn der Chip 175 reagiert auf die Funkabfrage nicht. Dies kann beispielsweise zum Nachweis der Echtheit und Unversehrtheit der Verpackungseinheit und der darin aufgenommenen Behälter dienen.

Gemäß einer weiter bevorzugten Ausführungsform ist der RuBee- oder RFID Chip 175 in Kombination mit weiteren Sensoren integriert, die wichtige Parameter des Transport- und Verpackungsbehälters 1 zeitabhängig überwachen können, welche wichtige Qualitäts- oder Echtheitseigenschaften der in dem Transport- und Verpackungsbehälter 1 aufbewahrten Behälter betreffen. Diese Qualitäts- oder Echtheitseigenschaften können periodisch aufgezeichnet und in einem dem Chip 175 oder Sensor zugeordneten Speicher abgelegt werden. Zur elektrischen Stromversorgung dieser elektronischen Bauelemente kann in dem Transport- und Verpackungsbehälter 1 eine netzunabhängige Spannungsversorgung vorgesehen sein, insbesondere eine Batterie mit geringen Abmessungen oder auch induktiv über eine kleine Leiterschleife. Als Sensoren sind gemäß der vorliegenden Anmeldung insbesondere angedacht:
- ein Feuchtigkeitssensor mit oder ohne Speicher (Data-Logging), der die in dem Transport- und Verpackungsbehälter vorherrschende Luftfeuchtigkeit periodisch misst und bei Bedarf aufzeichnet;
- ein Gassensor mit oder ohne Speicher (Data-Logging), der die Konzentration von in dem Transport- und Verpackungsbehälter vorhandenen Gasen, wie beispielsweise O₂, Ozon, CO₂ oder von Entkeimungsgase, wie z.B. Ethylenoxyd, Formaldehyd, misst und bei Bedarf aufzeichnet;
- ein Temperatursensor mit oder ohne Speicher (Data-Logging), der die in dem Transport- und Verpackungsbehälter vorherrschende Temperatur periodisch misst und bei Bedarf aufzeichnet;
- ein UV Sensor mit oder ohne Speicher (Data-Logging), der in den Transport- und Verpackungsbehälter eindringende UV-Strahlung periodisch misst und bei Bedarf aufzeichnet;
- ein Gammastrahlungs-, Elektronenstrahlen- oder Röntgenstrahlensensor mit oder ohne Speicher (Data-Logging), der in den Transport- und Verpackungsbehälter eindringende Strahlung periodisch misst und bei Bedarf aufzeichnet;

Die von den reibschlüssig wirkenden (klemmenden) Haltemitteln jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Gemäß weiteren Ausführungsformen kann die Haltekraft durch geeignete Auslegung der Haltemittel auch so bemessen sein, dass diese größer ist als übliche Kraft bei der Handhabung, Verarbeitung oder Behandlung der Behälter in einer Prozessanlage. Dadurch wird stets eine zuverlässige Halterung der Behälter gewährleistet. Gleichwohl können die Behälter gemäß bevorzugten weiteren Ausführungsformen der Erfindung gegen diese Haltekraft in den Öffnungen oder Aufnahmen verstellt werden, insbesondere axial vorgeschoben oder gedreht werden. Die hierfür erforderliche Kraft muss nur größer sein als die von den Haltemitteln ausgeübte Kraft.

Selbstverständlich kann die Haltestruktur (der Träger) im Sinne der vorliegenden Erfindung auch aus einem thermoplastischen, duroplastischen oder elastomeren Kunststoff ausgebildet sein, zumindest Abschnitte der Haltestruktur bzw. des Trägers mit einer reibreduzierenden Beschichtung versehen sind, um das Einführen und die Entnahme der Behälter zu erleichtern.

Gemäß weiteren Ausführungsform kann die Haltestruktur und/oder der Transportbehälter, oder Abschnitte davon, aus einem faserverstärkten Kunststoffe oder einem Kunststoff ausgebildet sein, dem zur Erhöhung seiner Wärmeleitfähigkeit Keramiken oder Metalle beigegeben beigegeben sind. Bekanntermaßen haben faserverstärkte Kunststoffe eine höhere Wärmeleitfähigkeit bis 0,9 W/(K m) bei Kohlenstofffasern. Werden den Kunststoffen Keramiken oder Metalle beigegeben, so wird die Wärmeleitfähigkeit weiter vergrößert. Es entstehen die sogenannten wärmeleitenden Kunststoffe. So wird eine Wärmeleitfähigkeit von 20 W/(K m) erreicht.

Wie dem Fachmann beim Studium der vorstehenden Beschreibung ohne Weiteres sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorliegenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Haltestruktur mit einer Mehrzahl von darin gehaltenen zylindrischen Behältern (2) für Substanzen für medizinische, pharmazeutische oder kosmetische Anwendungen, mit einem Träger (25; 134), der eine Mehrzahl von Aufnahmen (39) aufweist, in die die Behälter jeweils eingeführt sind, wobei die Aufnahmen sich in Längsrichtung der Behälter erstrecken, **durch gekennzeichnet, dass** die Aufnahmen von Seitenwänden ausgebildet sind, wobei die Seitenwände jeweils nicht mehrstückig ausgebildet sind, die Seitenwände Seitenwandabschnitte (4) der Behälter (2) in einer zweiten Stellung der Haltestruktur zumindest abschnittsweise klemmen, sodass sämtliche Behälter in der zweiten Stellung der Haltestruktur geklemmt gehalten sind, und
die Haltestruktur ausgelegt ist, sodass die Öffnungsweiten von sämtlichen Aufnahmen (39) der Haltestruktur zwischen der zweiten Stellung und einer ersten Stellung durch koordiniertes Verstellen der Seitenwände der Aufnahmen verstellbar sind, wobei die Behälter in der ersten Stellung frei oder mit geringem Kraftaufwand in die Aufnahmen einführbar sind.

2. Haltestruktur nach Anspruch 1, wobei die Aufnahmen (39) von plattenförmig und einstückig ausgebildeten Seitenwänden ausgebildet sind.

3. Haltestruktur nach Anspruch 1 oder 2, wobei in der zweiten Stellung der Haltestruktur Böden (3) oder untere Enden der Behälter von einer ersten Seite des Trägers her frei zugänglich sind und sämtliche Böden oder untere Enden der Behälter unter demselben Abstand zur Oberseite des Trägers (25) angeordnet sind, um gemeinsam eine Ebene aufzuspannen.

4. Haltestruktur nach einem der vorhergehenden Ansprüche, wobei die Aufnahmen einen vieleckigen Querschnitt aufweisen, insbesondere einen viereckigen oder sechseckigen Querschnitt, wobei die Klemmung der Behälter in der zweiten Stellung der Aufnahmen durch das Zusammenwirken von einander gegenüberliegenden Seitenwänden der Aufnahmen und der zylindrischen Seitenwand (4) der Behälter bewerkstelligt ist.

5. Haltestruktur nach Anspruch 4, wobei die Seitenwände (35, 38; 46a; 101, 103) der Aufnahmen aus einem elastischen Kunststoff ausgebildet oder mit einem solchen beschichtet oder versehen sind.

6. Haltestruktur nach einem der vorhergehenden Ansprüche, wobei der Träger (25) in der zweiten Stellung rechteckig oder quadratisch ist und die Haltestruktur so ausgelegt ist, dass die koordinierte Verstellbewegung eine Scherbewegung von einer ersten Stellung, in welcher der Träger in Draufsicht ein Parallelogramm ausbildet, in die zweite Stellung ist.

7. Haltestruktur nach einem der Ansprüche 1 bis 5, wobei die Aufnahmen aus einer Mehrzahl von identisch ausgebildeten und miteinander in einem Eingriff befindlichen Grundeinheiten (100) ausgebildet sind, wobei Seitenwände (101, 103, 107) von unmittelbar benachbarten Grundeinheiten (100) so miteinander zusammenwirken, dass diese für die koordinierte Verstellbewegung geradgeführt sind.

8. Haltestruktur nach Anspruch 7, wobei die Grundeinheiten von sich kreuzenden Seitenwänden ausgebildet werden, wobei zwei Seitenwände (101, 103) als Führungswände wirken, die geschlitzt ausgebildet sind und mit zumindest einem Langloch (105, 106) versehen sind, wobei in einem Schlitz der Führungswände jeweils Seitenwände (107) einer benachbarten Grundeinheit geradgeführt sind, die mit zumindest einem Vorsprung (108, 109) versehen sind, der in ein jeweils zugeordnetes Langloch (105, 106) einer benachbarten Grundeinheit eingreift.

9. Haltestruktur nach einem der vorhergehenden Ansprüche, wobei die Aufnahmen (39; 65)
an beiden Enden geöffnet ausgebildet sind, wobei am unteren Ende der Aufnahmen ein Haltesteg (47) zur Abstützung des in der jeweiligen Aufnahme abgestützten Behälters ausgebildet ist, oder
an beiden Enden geöffnet ausgebildet sind, wobei die Aufnahmen einen elastischen Steg (47) aufweisen, der einander gegenüberliegende Seitenwandabschnitte der Aufnahmen gegeneinander verspannt und diese zusammenzieht.

10. Haltestruktur nach einem der vorhergehenden Ansprüche, wobei Randabschnitte (150) der Haltestruktur (25) abnehmbar oder wegschwenkbar sind, um die Grundfläche der Haltestruktur insgesamt zu verringern.

11. Haltestruktur nach einem der vorhergehenden Ansprüche, wobei Stege oder Seitenwände zwischen den Aufnahmen eine unmittelbare Berührung von benachbarten Behältern, die darin aufgenommen sind, verhindern.

12. Transport- oder Verpackungsbehälter für eine Mehrzahl von Behältern (2) für Substanzen für medizinische, pharmazeutische oder kosmetische Anwendungen, umfassend einen kastenförmigen Behälter (10), **gekennzeichnet durch** eine in dem kastenförmigen Behälter (10) aufgenommene Haltestruktur (25) nach einem der vorhergehenden Ansprüche, in welcher die Mehrzahl von Behältern gehalten ist.

13. Transport- oder Verpackungsbehälter nach Anspruch 12, bestehend zumindest aus einem antimikrobiellen thermoplastischen, duroplastischen oder elastomeren Kunststoff, dem antimikrobiell wirkendes Pulver oder Glaspulver zugesetzt ist.

14. Transport- oder Verpackungsbehälter nach Anspruch 13, wobei das antimikrobiell wirkende Pulver oder Glaspulver dem Kunststoff ausschließlich oder hauptsächlich in den oberflächennahen Partien des Kunststoffes zugesetzt werden.

15. Transport- oder Verpackungsbehälter nach einem der Ansprüche 12 bis 14, weiterhin umfassend eine Schutz- oder Verpackungsfolie (130), die auf einen oberen Rand (15) des kastenförmigen Behälters (10) aufgeklebt ist, sowie ein Identifikations- oder Nachverfolgungsmittel (175) zum Identifizieren oder Nachverfolgen des Transport- oder Verpackungsbehälters oder der Haltestruktur (25) mit den darin gehaltenen Behältern, wobei das Identifikations- oder Nachverfolgungsmittel (175) ein RFID-Chip oder ein RuBee-Chip zum berührungslosen Auslesen von Information ist, der so mit der Haltestruktur (25) und/oder dem Transport- oder Verpackungsbehälter (1) und/oder der Schutz- oder Verpackungsfolie (130) verbunden ist, dass dieser beim Öffnen des Transport- oder Verpackungsbehälters (1) oder bei Entnahme oder Manipulation der Haltestruktur (25) oder der darin aufgenommenen Behälter (2) zerstört wird.

16. Transport- oder Verpackungsbehälter nach Anspruch 15, wobei weiterhin zumindest ein Sensor vorgesehen ist, um Parameter des Transport- oder Verpackungsbehälters zeitabhängig zu überwachen oder zeitabhängig zu überwachen und aufzuzeichnen.

## Claims

1. A supporting structure with a plurality of cylindrical containers (2) for substances for medical, pharmaceutical or cosmetic applications supported therein, comprising a carrier (25; 134) having a plurality of receptacles (39) into which the containers are respectively inserted, wherein the receptacles extend in the longitudinal direction of the containers,
**characterized in that** the receptacles are formed by side walls, wherein
the respective side walls are not formed by a plurality of pieces,
the side walls clamp side wall portions (4) of the containers (2) at least partially in a second position of the supporting structure so that all containers are held clamped in the second position of the supporting structure, and
the supporting structure is configured such that the opening widths of all receptacles (39) of the supporting structure can be adjusted by a coordinated adjustment of the side walls of the receptacles between the second position and a first position, wherein the containers can be inserted into the receptacles freely or with minimal forces in the first position.

2. The supporting structure of claim 1, wherein the receptacles (39) are formed by plateshaped and single-piece side walls.

3. The supporting structure of claim 1 or 2, wherein in the second position of the supporting structure bottoms (3) or bottom ends of the containers are freely accessible from a first side of the carrier and wherein all bottoms or bottom ends of the containers are disposed under the same distance to the upper side of the carrier (25) for jointly spanning a plane.

4. The supporting structure of any of the preceding claims, wherein the receptacles have a polygonal cross section, in particular a quadrangular or hexagonal cross section, wherein the clamping of the containers in the second position of the receptacles is accomplished by the cooperation of opposite side walls of the receptacles and of the cylindrical side wall (4) of the containers.

5. The supporting structure of claim 4, wherein the side walls (35, 38; 46a; 101, 103) of the receptacles are formed of a flexible plastic or coated or provided with such a flexible plastic.

6. The supporting structure of any of the preceding claims, wherein the carrier (25) is rectangular or square-shaped in the second position and wherein the supporting structure is configured such that the coordinated adjustment is a shearing movement from a first position, in which the carrier forms a parallelogram if viewed in a plan view, to the second position.

7. The supporting structure of any of claims 1 to 5, wherein the receptacles are formed by a plurality of base units (100) which are identical and are in engagement with each other, wherein side walls (101, 103, 107) of directly adjacent base units (100) interact with each other such that they are linearly guided for said coordinated adjustment.

8. The supporting structure of claim 7, wherein the base units are formed by intersecting side walls, wherein two side walls (101, 103) act as guiding walls that are formed with a slot and are provided with at least one elongated hole (105, 106), wherein respective side walls (107) of an adjacent base unit are linearly guided in a slot of the guiding walls, which are provided with at least one projection (108, 109) which engages in a respective associated elongated hole (105, 106) of an adjacent base unit.

9. The supporting structure of any of the preceding claims, wherein the receptacles (39; 65)
are open at both ends, wherein a retaining web (47) is formed at the bottom end of the receptacles for supporting the container supported in the respective receptacle, or
are open at both ends, wherein the receptacles comprise an elastic web (47) which biases opposite side wall portions of the receptacles against each another and pulls them together.

10. The supporting structure of any of the preceding claims, wherein rim portions (150) of the supporting structure (25) can be removed or pivoted away to reduce the base area of the supporting structure.

11. The supporting structure of any of the preceding claims, wherein webs or side walls between the receptacles prevent a direct collision of adjacent containers accommodated therein.

12. A transport or packaging container for a plurality of containers (2) for substances for medical, pharmaceutical or cosmetic applications, comprising a box-shaped container (10), **characterized by** a supporting structure (25) according to any of the preceding claims accommodated in the box-shaped container (10) in which a plurality of containers are supported.

13. The transport or packaging container of claim 12, consisting of at least one antimicrobial thermoplastic, thermosetting or elastomeric plastic to which an antimicrobial powder or glass powder is added.

14. The transport or packaging container of claim 13, wherein the antimicrobial powder or glass powder is added to the plastic material solely or mainly in the portions of the plastic near a surface thereof.

15. The transport or packaging container of any of claims 12 to 14, further comprising a protective or packaging foil (130) which is bonded to an upper rim (15) of the box-shaped container (10), and an identification or tracking means (175) for identifying or tracking the transport or packaging container or the supporting structure (25) with the containers supported therein, wherein the identification or tracking means (175) is an RFID chip or a RuBee chip for reading out information in a contactless manner, which is coupled to the supporting structure (25) and/or to the transport or packaging container (1) and/or to the protective or packaging foil (130) in such a manner that it is destroyed upon opening of the transport or packaging container (1) or upon removal or manipulation of the supporting structure (25) or of the containers (2) accommodated therein.

16. The transport or packaging container of claim 15, further comprising at least one sensor for monitoring or recording parameters of the transport or packaging container as a function of time.

## Revendications

1. Une structure de support pour le maintien d'une pluralité de récipients cylindriques (2) pour des substances destinées à des applications cosmétiques, pharmaceutiques ou médicales, comprenant un support (25; 134) comportant une pluralité de réceptacles (39) dans lesquels les récipients sont respectivement insérés, dans laquelle les réceptacles se prolongent dans la direction longitudinale des récipients,
**caractérisé en ce que** les réceptacles sont formés par des parois latérales, dans lesquels
les parois latérales ne sont pas formés d'une pluralité de pièces,
les parois latérales s'attachent à des parties le parois latérales (4) des récipients (2) au moins partiellement dans une seconde position de la structure de support de telle façon que tous les récipients sont tenus attachés dans la seconde position de la structure de support, et
la structure de support est configurée de telle façon que les largeurs d'ouverture de tous les réceptacles (39) de la structure de support peut être ajustée au moyen d'un déplacement coordonné des parois latérales des réceptacles entre la seconde position et une première position, dans laquelle les récipients peuvent être insérés dans la première position à l'intérieur des réceptacles, librement ou par des efforts minimes.

2. La structure de support de la revendication 1, dans laquelle les réceptacles (39) sont formés par une plaque formée et des parois latérales en une pièce.

3. La structure de support de la revendication 1 ou 2, dans laquelle, dans la seconde position de la structure de support, des éléments inférieurs (3) ou des extrémités inférieures des récipients sont librement accessible depuis un premier côté du support et dans lequel tous les éléments inférieurs ou les extrémités inférieures des récipients sont disposés à une même distance du côté supérieure du support (25) pour couvrir conjointement un plan.

4. La structure de support de l'une quelconque des revendications précédentes, dans laquelle les réceptacles présentent une section transversale polygonale, en particulier une section carrée ou hexagonale, dans laquelle le serrage des récipients dans la deuxième position des réceptacles est réalisé par l'interaction des parois latérales opposées des réceptacles et la paroi latérale cylindrique (4) des récipients.

5. La structure de support de la revendication 4, dans laquelle les parois latérales (35, 38 ; 46a; 101, 103) des réceptacles sont formées de plastique flexible ou recouvert ou dotées d' un tel plastique.

6. La structure de support de l'une quelconque des revendications précédentes, dans laquelle le support (25) est rectangulaire ou de forme carrée dans la seconde position et dans lequel la structure de support est configurée de telle façon que le déplacement coordonné est un mouvement de cisaillement à partir d'une première position dans laquelle le support forme un parallélogramme suivant une vue plane, vers la seconde position.

7. La structure de support de l'une quelconque des revendications 1 à 5, dans laquelle les réceptacles sont formés d'une pluralité d'unités de base (100) identique et en prise l'une avec l'autre, dans laquelle les parois latérales (101, 103, 107) d'unités de base directement adjacentes (100) interagissent l'une avec l'autre de telle manière qu'elles sont guidés linéairement pour ledit déplacement coordonné.

8. La structure de support de la revendication 7, dans laquelle les unités de base sont formées par les parois latérales qui se croisent, dans laquelle deux parois latérales (101, 103) agissent en tant que parois de guidage qui sont formées avec une fente et au moins un trou allongé (105, 106), dans laquelle des parois latérales respectives (107) d'une unité de base adjacente sont guidés linéairement au sein d'une fente des parois latérales, qui sont pourvues d'au moins une saillie (108, 109) qui est en prise dans un trou allongé associé respectif (105, 106) d'une unité de base adjacente.

9. La structure de support de l'une quelconque des revendications précédentes, dans laquelle les réceptacles (39 ; 65)
sont ouverts aux deux extrémités, dans laquelle une bande de retenue (47) est formée à l'extrémité inférieure des réceptacles pour soutenir le récipient supporté dans le réceptacle respectif, ou
sont ouverts aux deux extrémités, dans laquelle les réceptacles comportent une bande élastique (47) venant contraindre des portions de parois latérales opposées des réceptacles, l'une contre l'autre, et les poussent l'une vers l'autre.

10. La structure de support de l'une quelconque des revendications précédentes, dans lequel des parties de bord (150) de la structure de support (25) sont amovibles ou peuvent être écartées par pivotement pour réduire l'encombrement de la structure de support.

11. La structure de support de l'une quelconque des revendications précédentes, dans lequel les bandes ou les parois latérales entre les réceptacles empêchent un contact direct entre des récipients adjacents qui y sont maintenues.

12. Un récipient de transport ou d'emballage pour une pluralité de récipients (2) pour des substances destinées à des applications cosmétiques, pharmaceutiques ou médicales, comprenant un récipient en forme de boîte (10),
**caractérisé par** une structure de support (25) selon l'une quelconque des revendications précédentes disposée au sein du récipient en forme de boîte (10) dans lequel sont maintenus une pluralité de récipients.

13. Le récipient de transport ou d'emballage de la revendication 12, consistant en au moins une matière thermoplastique antimicrobienne, une matière plastique thermodurcissable ou élastomère, à laquelle est ajoutée une poudre antimicrobienne ou une poudre de verre.

14. Le récipient de transport ou d'emballage de la revendication 13, dans lequel la poudre antimicrobiennes ou la poudre de verre est ajoutée à la matière plastique uniquement ou principalement dans les parties superficielles du plastique.

15. Le récipient de transport ou d'emballage de l'une quelconque des revendications 12 à 14, comprenant en outre un film protecteur ou d'emballage (130), collé sur un bord supérieur (15) du récipient en forme de boîte (10), et des moyens d'identification ou de suivi (175) pour l'identification ou le suivi du récipient de transport ou d'emballage ou de la structure de support (25) dans laquelle sont maintenus les récipients, dans lequel les moyens d'identification ou de suivi (175) consistent en une puce RFID ou une puce RuBee pour une lecture d'information sans contact , qui est couplée à la structure de support (25) et/ou au récipient de transport ou d'emballage (1) et/ou au film protecteur ou d'emballage (130) de telle façon qu'il est détruit dès l'ouverture du récipient de transport ou d'emballage (1) ou lors de l'enlèvement ou de la manipulation de la structure de support (25) ou des récipients (2) qui y sont logés.

16. Le récipient de transport ou d'emballage de la revendication 15, comprenant en outre au moins un capteur pour suivre ou enregistrer des paramètres du récipient de transport ou d'emballage en fonction du temps.
